(19) Europäisches Patentamt
European Patent Office
Office européen des brevets

(11) EP 1 014 934 B1

# (12) FASCICULE DE BREVET EUROPEEN

(45) Date de publication et mention de la délivrance du brevet:
**16.01.2002 Bulletin 2002/03**

(21) Numéro de dépôt: **98943977.3**

(22) Date de dépôt: **14.09.1998**

(51) Int Cl.$^7$: **A61K 7/48**, A61K 7/42, A61K 7/06

(86) Numéro de dépôt international:
**PCT/FR98/01958**

(87) Numéro de publication internationale:
**WO 99/13856 (25.03.1999 Gazette 1999/12)**

## (54) UTILISATION D'AU MOINS UN EXTRAIT DU GENRE CHRYSANTHEMUM POUR FAVORISER LA PIGMENTATION DE LA PEAU ET/OU DES CHEVEUX

VERWENDUNG VON MINDESTENS EINEN CHRYSANTHEMUM GATTUNGS EXTRAKTEN ZUR FÖRDERUNG DER HAUT- UND/ODER DER HAAREPIGMENTIERUNG

USE OF AT LEAST AN EXTRACT OF THE GENUS CHRYSANTHEMUM FOR STIMULATING SKIN AND/OR HAIR PIGMENTATION

(84) Etats contractants désignés:
**AT BE CH CY DE DK ES FI FR GB GR IE IT LI LU MC NL PT SE**

(30) Priorité: **18.09.1997 FR 9711648**

(43) Date de publication de la demande:
**05.07.2000 Bulletin 2000/27**

(73) Titulaire: **L'OREAL**
**75008 Paris (FR)**

(72) Inventeurs:
- **MARTIN, Richard**
  **F-37210 Rochecorbon (FR)**
- **BELCOUR-CASTRO, Béatrice**
  **F-37520 La Riche (FR)**

(74) Mandataire: **Galup, Cédric Olivier Nicolas et al**
**L'OREAL Départment Propriété Industrielle 6, rue Bertrand Sincholle**
**92585 Clichy Cedex (FR)**

(56) Documents cités:
**US-A- 4 911 925**

- **DATABASE WPI Week 9726 Derwent Publications Ltd., London, GB; AN 97-281465 XP002062463 & CN 1 102 340 A (LIU), 10 mai 1994**
- **DATABASE WPI Week 9543 Derwent Publications Ltd., London, GB; AN 95-328816 XP002062464 & CN 1 094 278 A (WANG) cité dans la demande**
- **DATABASE WPI Week 8714 Derwent Publications Ltd., London, GB; AN 87-099079 XP002062465 & JP 62 048611 A (SHISEIDO CO) cité dans la demande**
- **PATENT ABSTRACTS OF JAPAN vol. 7, no. 248 (C-193) & JP 58 135803 A (NORIKO SUGANO)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 014 934 B1

## Description

L'invention concerne l'utilisation d'au moins un extrait d'au moins un végétal du genre Chrysanthemum dans une composition pour favoriser la pigmentation de la peau et/ou des cheveux.

La couleur des cheveux et de la peau humaine est fonction de différents facteurs et notamment des saisons de l'année, de la race, du sexe et de l'âge. Elle est principalement déterminée par la concentration dans les kératinocytes de la mélanine produite par les mélanocytes. Les mélanocytes sont les cellules spécialisées qui par l'intermédiaire d'organelles particuliers, les mélanosomes, synthétisent la mélanine.

La synthèse de la mélanine ou mélanogénèse est particulièrement complexe et fait intervenir schématiquement les principales étapes suivantes :

Tyrosine → Dopa → Dopaquinone → Dopachrome → Mélanine

La tyrosinase (monophénol dihydroxyl phénylalanine : oxygen oxydoreductase / EC 1,14,18,1) est l'enzyme essentielle intervenant dans cette suite de réactions. Elle catalyse notamment la réaction de transformation de la tyrosine en Dopa (dihydroxyphénylalanine) et la réaction de transformation de la Dopa en Dopaquinone.

Dans l'épiderme, le mélanocyte est impliqué dans l'unité mélanique épidermique qui comporte un mélanocyte entouré d'environ 36 kératinocytes voisins. Tous les individus, sans distinction de phototype, ont approximativement le même nombre de mélanocytes pour une zone cutanée donnée. Les différences ethniques, en terme de pigmentation, ne sont pas dues au nombre de mélanocytes, mais aux propriétés de leurs mélanosomes. Les mélanosomes sont agrégés en complexes et sont de petites tailles. Ce sont des organelles hautement spécialisées dont l'unique fonction est la production de mélanine. ils naissent du réticulum endoplasmique sous formes de vacuoles sphériques appelées prémélanosomes. Les prémélanosomes contiennent un substrat protéinique amorphe, mais pas d'enzymes mélanogènes. Au cours de la maturation du prémélanosome, le substrat amorphe s'organise en une structure fibrillaire orientée dans l'axe longitudinal du mélanosome. On distingue quatre stades du développement du mélanosome correspondant à l'intensité de la mélanisation. La mélanine est déposée uniformément sur le réseau fibrillaire interne du mélanosome et l'opacité de l'organelle augmente jusqu'à saturation. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes.

Bien que le taux de mélanine varie d'une population à une autre, la quantité de tyrosinase ne varie pas significativement et le taux d'ARN messagers de la tyrosinase est identique dans des peaux blanches ou noires. Les variations dans la mélanogénèse sont donc dues à des variations soit de l'activité de la tyrosinase soit de la capacité des kératinocytes à phagocyter les mélanosomes.

On sait que dans la plupart des populations la coloration brune de la peau et le maintien d'une coloration constante de la chevelure sont des aspirations importantes.

Il existe par ailleurs des maladies de la pigmentation comme par exemple le vitiligo qui est une maladie auto-immune qui se caractérise par l'apparition de plaques blanches sur la peau liées à un défaut de pigmentation.

Il existe donc un réel besoin de produit facilitant et/ou améliorant la pigmentation de la peau et/ou des cheveux.

De nombreuses solutions ont été proposées dans le domaine de la coloration artificielle par apport de colorants exogènes sensés donner à la peau et/ou les cheveux une coloration la plus proche possible de ce qu'elle est naturellement ou dans le domaine de la coloration naturelle par stimulation des voies naturelles de pigmentation.

Par exemple, il a été proposé dans les documents WO-A-9517161, WO-9511003, WO-A-9501773, WO-A-9404674, WO-A-9404122, EP-A-585018, WO-A-9310804, WO-A-9220322 ou WO-A-9107945 des solutions aussi variées que des compositions contenant un inhibiteur de phosphodiestérases, l'utilisation de prostaglandines, de fragments d'ADN ou encore de dérivés de la tyrosine.

D'excellents résultats sont certes obtenus par les solutions proposées dans l'art antérieur, mais les composés utilisés présentent souvent des effets secondaires non négligeables ou sont des mélanges complexes qui ne présentent pas de spécificité.
La découverte de substances ayant un effet sur la pigmentation de la peau et/ou des cheveux sans présenter d'effets secondaires gênants reste un objectif majeur de la recherche.

La demanderesse a maintenant découvert qu'au moins un extrait d'au moins un végétal du genre Chrysanthemum a un effet activateur de la mélanogénèse.

Les végétaux du genre Chrysanthemum sont utilisés dans l'art antérieur dans des compositions ayant des propriétés dépigmentantes (JP07025745, JP07025746), des compositions favorisants la croissance des cheveux et/ou luttant contre la chute des cheveux (FR2659014, EP569667, DE4330597, DE4312109, JP8081336, JP84154598, JP02048514), des compositions antipelliculaires (KR9006824, JP62153211) ou des compositions pour le maintien de

la souplesse, de l'hydratation et de la brillance de la peau (JP62048611).
Les demandes de brevet CN1094278 et CN1094279 ont pour objet des compositions pour le traitement naturel des cheveux. Ces compositions présentent une variété de propriétés (embellissement des cheveux, fixation, promotion de la circulation sanguine et de la croissance des cheveux, bactéricide, antidémangeaison, antipelliculaire) parmi lesquelles une action anticheveux blancs est citée. Ces compositions sont en fait un mélange de 2 composants constitués chacun d'extraits d'au moins 9 plantes, sans qu'il puisse être attribuée à l'une plutôt qu'à une autre une des nombreuses propriétés citées.

A la connaissance de la demanderesse il n'a jamais été revendiqué l'utilisation à titre de principe actif d'un extrait de végétal du genre Chrysanthemum pour favoriser la pigmentation de la peau et/ou des cheveux.

L'invention a donc pour objet l'utilisation dans une composition cosmétique ou pour la préparation d'une composition pharmaceutique, à titre de principe actif, d'une quantité suffisante d'au moins un extrait d'au moins un végétal du genre Chrysanthemum, cet extrait ou la composition étant destiné à augmenter la pigmentation de la peau et/ou des cheveux.

L'invention concerne également l'utilisation dans une composition cosmétique ou pour la préparation d'une composition pharmaceutique, à titre de principe actif, d'une quantité suffisante d'au moins un extrait d'au moins un végétal du genre Chrysanthemum pour favoriser la mélanogénèse.

L'extrait d'au moins un végétal du genre Chrysanthemum peut être tout extrait préparé à partir de tout matériel végétal issu d'un végétal du genre Chrysanthemum.
La composition peut contenir au moins un extrait d'au moins un végétal du genre Chrysanthemum obtenu à partir de matériel issu d'un végétal cultivé *in vivo* ou issu de culture *in vitro*.

Par culture *in vivo* on entend toute culture de type classique c'est à dire en sol à l'air libre ou en serre, ou encore hors sol.

On peut ainsi utiliser par exemple selon l'invention un extrait de différentes parties du végétal, feuilles, fleurs, tiges, racines, cellules indifférenciées, seules ou en mélange, que le végétal soit cultivé *in vivo* ou *in vitro*.

La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo*.

Par culture *in vitro*, on entend l'ensemble des techniques connues de l'homme du métier qui permet de manière artificielle l'obtention d'un végétal ou d'une partie d'un végétal.

De préférence on utilise un extrait obtenu à partir de matériel végétal cultivé *in vivo* et encore plus préférentiellement un extrait obtenu à partir de feuilles de végétaux du genre Chrysanthemum cultivées *in vivo*.

Le genre Chrysanthemum appartient à la famille des Compositae qui lui même appartient à l'ordre des Asteracae (ou Astérales).
Le genre Chrysanthemum comporte environs 200 espèces originaires d'Europe et d'Asie, parmi lesquelles on peut citer *Chrysanthemum hortorum, Chrysanthemum morifolium, Chrysanthemum coronarium, Chrysanthemum myconis, Chrysanthemum sagitum, Chrysanthemum indicum,* ou encore *Chrysanthemum segetum*

Préférentiellement, selon l'invention on utilise un végétal issus de l'espèce *Chrysanthemum morifolium*.
L'espèce *Chrysanthemum morifolium* compte de nombreuses variétés parmi lesquelles on peut citer la variété *sinense*, préférentiellement utilisée selon l'invention.

Par extrait d'au moins un végétal du genre Chrysanthemum, on entend aussi bien un mélange brut de parties du végétal grossièrement réduites en morceaux et du solvant d'extraction que des préparations élaborés des principes actifs solubilisés lors de l'extraction. Un extrait particulièrement utilisable selon l'invention est réalisé par le broyage fin de parties du végétal suivi d'une macération dans le solvant d'extraction et finalement d'une filtration. Bien évidemment l'extrait selon l'invention peut être chacun des extraits ainsi obtenus utilisé seul ou en mélange avec l'un ou plusieurs des autres extraits.

Toute méthode d'extraction connue de l'homme du métier peut être utilisée selon l'invention.
On peut en particulier citer les extraits aqueux, alcooliques, notamment éthanoliques, les extraits hydroalcooliques.
Quelle que soit la forme de l'extrait que l'on désire utiliser, les techniques utilisées pour l'obtenir sont celles généralement décrites dans l'art antérieur et bien connues de l'homme du métier.

On peut également utiliser un extrait préparé par la méthode décrite dans la demande de brevet français n° 95-02379.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape.

Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélangée dans un solvant approprié avant utilisation.
Préférentiellement selon l'invention on utilise un extrait aqueux.

Des modes de préparation d'extrait détaillés utilisables selon l'invention sont donnés par ailleurs dans les exemples.

La quantité d'extrait d'au moins un végétal du genre Chrysanthemum contenue dans la composition de l'invention est bien entendu fonction de l'effet recherché et de la forme de l'extrait utilisé dans la composition. Elle peut donc varier dans une large mesure.

Pour donner un ordre de grandeur, quelle que soit sa nature, la composition peut contenir un extrait d'au moins un végétal du genre Chrysanthemum en une quantité représentant de 0,01% à 15% du poids total de la composition et préférentiellement en une quantité représentant de 1% à 5% du poids total de la composition.

Les compositions selon l'invention sont essentiellement destinées à augmenter la pigmentation de la peau et/ou des cheveux et/ou à stimuler la mélanogénèse.
Quelle que soit sa nature, la composition de l'invention est essentiellement appliquée sur la peau (sur toute zone cutanée du corps) et/ou les cheveux.

Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose$^{R}$ 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention, la composition peut comprendre d'autres agents actifs destinés notamment à la prévention et/ou au traitement des affections cutanées. Parmi ces agents actifs, on peut citer à titre d'exemple :

- les agents diminuant la différenciation et/ou la prolifération cutanée tels que l'acide rétinoïque et ses isomères, le rétinol et ses esters, la vitamine D et ses dérivés, les oestrogènes tels que l'oestradiol ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides $\alpha$- et $\beta$-hydroxycarboxyliques ou $\beta$-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'$\alpha$-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les agents favorisant la pigmentation de la peau et/ou des cheveux comme par exemple les substrats d'au moins une enzyme présentant une activité tyrosinase comme par exemple la tyrosine ou ses dérivés ou la dihydroxy-3,4 phényl $\alpha$-alanine (DOPA), les dérivés de pyrimidine 3-oxyde, substitué en 6 comme par exemple ceux décrits dans la demande de brevet de la demanderesse déposée en France sous le numéro 96-11316 répondant à la formule générale (I)

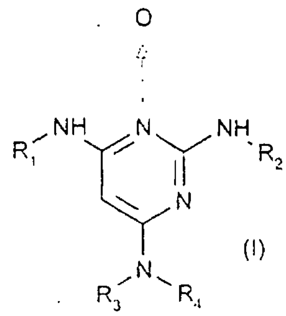

(I)

dans laquelle

$R_1$ et $R_2$ identiques ou différents, représentent un atome d'hydrogène ou un radical alkyle en $C_1$ - $C_{12}$;
$R_3$ et $R_4$ identiques ou différents, représentent un radical alkyle en $C_1$ - $C_{12}$, ou pris ensemble peuvent former avec l'atome d'azote auquel ils sont reliés un hétérocycle ;
étant entendu que lorsque R3 et R4 pris ensemble forment un cycle pipéridino alors au moins l'un des radicaux R1 ou R2 doit être différent d'un atome d'hydrogène parmi lesquels on peut citer particulièrement :

le 2-amino-4-propylamino-6-diméthylamino pyrimidine 3-oxyde,
le 2 amino-4-méthylamino-6-pipéridino pyrimidine 3-oxyde,
le 2,4 bis-propylamino-6-diméthylamino pyrimidine 3-oxyde,
le 2,4 bis-propylamino-6-pipéridino pyrimidine 3-oxyde,
le 2,4 bis-méthylamino-6-diméthylamino pyrimidine 3-oxyde,
le 2,4 bis-éthylamino-6-diméthylamino pyrimidine 3-oxyde ;
les extraits d'origine bactérienne comme par exemple les extraits de bactéries filamenteuses non photosynthétiques comme par exemple les extraits de *Vitreoscilla filiformis*.

Ainsi, la composition selon l'invention peut comprendre également au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques , les agents modulant la différenciation et/ou la prolifération cellulaire et/ou les agents favorisant la pigmentation de la peau et/ou des cheveux.

On peut également envisager que la composition soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

L'invention a également pour objet un procédé de traitement cosmétique pour augmenter la pigmentation de la peau et/ou des cheveux tel que l'on applique sur la peau et/ou les cheveux une composition cosmétique comprenant au moins un extrait d'au moins un végétal du genre Chrysanthemum dans un milieu cosmétiquement acceptable.

Par milieu cosmétiquement acceptable, on entend un milieu compatible avec la peau, les muqueuses, les ongles, les cheveux.

On va maintenant donner à titre d'illustration des exemples qui ne sauraient limiter en aucune façon la portée de l'invention.

Exemple 1 : Préparation d'un extrait de *Chrysanthemum morifolium* variété *sinense* :

Des feuilles de plants de *Chrysanthemum morifolium* variété *sinense* cultivés sous serre sont prélevées et séchées durant 48 heures dans une étuve ventilée à une température de 45°C.
Les feuilles séchées sont ensuite réduites en poudre par broyage sur un broyeur à couteau du type Culatti.

La poudre obtenue est tamisée sur une grille dont les trous ont un diamétre de 1 mm.
C'est cette poudre tamisée qui est utilisée pour la préparation de l'extrait.

Protocole 1 :

La poudre est mélangée à un solvant d'extraction aqueux constitué par du milieu de culture cellulaire DMEM/F 12,3 : 1 vendu par la société Life Technologies, à raison d'une concentration de 5 grammes de poudre sèche pour 100 ml de solvant. Le mélange est maintenu sous agitation pendant 4 heures à température ambiante. Le mélange

est alors centrifugé à 1000 tours /minute pendant 8 minutes et le surnageant est prélevé et soumis à deux cycles de centrifugations/prélèvement identiques.
Le dernier surnageant prélevé est filtré sur filtre 0,22 µm de type Millipore en conditions aseptiques afin d'être stérilisé et conservé à une température de 4°C. jusqu'à utilisation.

Protocole 2 :

La poudre est mélangée à de l'eau déminéralisée stérile ayant un pH de 6,5 à raison d'une concentration de 2,5 grammes de poudre sèche pour 100 ml d'eau. Le mélange est maintenu sous agitation pendant 30 minutes à température ambiante. Le mélange est alors filtré sur membranes GFD vendues par la société Whatmann ayant une porosité de 0,7µm. Le filtrat obtenu est alors filtré sur filtre 0,22 µm de type Nalgene en conditions aseptiques afin d'être stérilisé et conservé à une température de 4°C. jusqu'à utilisation.

Protocole 3 :

Le protocole précédent est réalisé en remplaçant l'eau par un solvant d'extraction aqueux constitué par du milieu de culture cellulaire DMEM/F 12,3 : 1 vendu par la société Life Technologies.

Protocole 4 :

L'extrait obtenu au protocole 2 est lyophilisé à 30°C après congélation à -20°C.
La poudre obtenue est utilisée directement.

Exemple 2 : Mesure de l'effet modulateur de la mélanogénèse de l'extrait obtenu par le protocole 2 de l'exemple 1 :

L'effet modulateur sur la mélanogénèse de l'extrait obtenu par le protocole 2 de l'exemple 1 a été testé sur co-cultures de kératinocytes / mélanocytes humains normaux selon la méthode décrite dans le brevet FR95 06491. Le taux de la synthèse de mélanine est évalué par l'incorporation de thiouracile marquée au $C^{14}$. La synthèse des protéines, dosée par l'incorporation de leucine tritiée, est prise comme indicateur de cytotoxicité et de prolifération.

MATERIEL ET METHODES

- Cultures cellulaires :

Les kératinocytes humains normaux (NHK) et les mélanocytes humains normaux (NHM) sont cultivés à partir de peau de prépuce. Les deux types de cellules sont amplifiés et stockés congelés. Huit jours avant le test, chacun des types cellulaires est remis en culture dans du milieu KGM de la société Gibco pour les kératinocytes (NHK) et dans le milieu M2 du Dr Olsson pour les mélanocytes (NHM) (Olsson, M.J. et al.., Lancet (1992) 340, 981). Les milieux sont changés tous les deux jours.
Quarante huit heures avant confluence, le milieu KGM des NHK est remplacé par un milieu de différenciation (DMEM/F12 3:1, sérum de veau 10%, EGF 10ng/ml, hydrocortisone 0.4µg/ml, insuline 5µg/ml).

- Extrait végétal

L'extrait végétal de l'exemple 1 (protocole 2) est testé aux concentrations de 1.25 $10^{-4}$, 2.5 $10^{-4}$, 5 $10^{-4}$, 1.25 $10^{-3}$, 2.5 $10^{-3}$, 5 $10^{-3}$ %.

- Modulation de la mélanogénèse

250 000 kératinocytes humains normaux et 80 000 mélanocytes humains normaux sont mélangés et ensemencés par puits de plaques de 24 puits (type Costar) et cultivés trois jours dans le milieu de différenciation. Pendant les trois jours suivants, le milieu de culture est remplacé quotidiennement par le milieu test défini (DMEM/F12 3:1, facteur de croissance épidermique (EGF) 10 ng/ml, facteur de croissance fibroblastique de type β (βFGF) 10 ng/ml) contenant 0,037MBq/ml (1 µCi/ml) de thiouracile marquée au $C^{14}$.
Les témoins suivants sont réalisés :

- culture contrôle: Pas de produit à tester ;
- témoin positif de stimulation de la mélanogénèse : 1 mM tyrosine ;

- témoin positifs d'inhibition de la mélanogénèse : 0,5 mM d'acide kojique.

La radioactivité totale incorporée dans les protéines est estimée par l'incorporation de leucine tritiée. La veille de la prise 0,037MBq/ml (1 µCi/ml) de leucine tritiée est ajouté au milieu test.
Après une nuit, les cellules sont rincées en tampon phosphate. Les protéines sont précipitées à l'aide d'acide trichloracétique (TCA) à 5% et lavées afin d'éliminer la radioactivité libre. Les protéines sont incubées pendant la nuit à 40°C à l'aide d'une solution de protéinase K à 100 µg/ml dans du tampon Tris HCl-triton-EDTA. 50 µl d'extrait total sont prélevés et transférés dans une plaque 24 puits (Wallac) et 500 µl de liquide scintillant (Optiphase « Supermix ») sont ajoutés.
Le reste de l'extrait, soit 950 µl, est filtré sur filtre DEAE Filtermat. Après rinçage, le filtre recouvert du scintillant solide « Meltilex » est transféré dans une plaque.
La radioactivité est comptée à l'aide du compteur Wallac. Les résultats sont exprimés en pourcentage du témoin selon la formule:

$$\frac{(14CP / 3HP) - (14CT / 3HT)}{(14CT / 3HT)} \text{ X } 100$$

dans laquelle:

$^{14}$CP est la moyenne des désintégrations par minute (dpm) thiouracile $^{14}$C sur 3 puits similaires traités par un produit (P) ;
$^{3}$HP est la moyenne des dpm leucine $^{3}$H correspondant ;
$^{14}$CT est la moyenne des dpm thiouracile $^{14}$ C sur 3 puits similaires témoin (T) ;
$^{3}$HT est la moyenne des dpm leucine $^{3}$H correspondant.

RESULTATS:

| Produits | $^{3}$HLeu. (%/Témoin) | $^{14}$CThioU. (%/Témoin) |
|---|---|---|
| Tyrosine 1 mM | - 2 | + 118 |
| Extrait 5.10$^{-4}$ % | - 1 | + 92 |
| Extrait 2.5 10$^{-3}$% | + 5 | + 502 |
| Extrait 5 10$^{-3}$ % | - 13 | + 932 |
| Acide kojique 0.5 mM | + 4 | - 47 |

CONCLUSIONS :

Ce produit est non cytotoxique aux concentrations testées (variation non significative de l'incorporation de $^{3}$HLeu.) et induit la synthèse: de mélanine à partir de 1.25 10$^{-3}$ % (augmentation de l'incorporation de $^{14}$CThioU).

Exemple 3 : Exemples de compositions contenant au moins un extrait d'au moins un végétal du genre Chrysanthemum.

Ces compositions sont obtenues par les techniques habituelles couramment utilisées en cosmétique ou en pharmacie.

| Crème dermique : | |
|---|---|
| Extrait de l'exemple 1, protocole 1 | 5,000 g |
| Ceteareth 30 | 7,000 g |
| Stéarate de glycéryle | 2,000 g |
| Alcool cétylique | 1,500 g |
| Polydiméthylsiloxane | 1,500 g |
| Huile de paraffine | 15,000 g |
| Glycérine codex pure | 20,000 g |

(suite)

| Crème dermique : | |
|---|---|
| Conservateurs q.s.<br>Eau déminéralisée q.s.p. | 100,000 g |
| Lotion dermique à pulvériser : | |
| Extrait de l'exemple 1, protocole 2<br>Dihydroxy-3,4 phényl α-alanine (DOPA)<br>Ethanol<br>Eau déminéralisée q.s.p. | 10,000 g<br>0,200 g<br>30,000 g<br>100,000 g |
| Lotion pour les cheveux : | |
| Extrait de l'exemple 1, protocole 4<br>Tyrosine<br>Propylène glycol<br>Alcool éthylique<br>Eau qsp | 0,500 g<br>1,000 g<br>30,000 g<br>40,500 g<br>100,000 g |

On applique cette lotion sur le cuir chevelu, une à deux fois par jour, à raison d''1 ml par application.

| Lotion épaissie : | |
|---|---|
| Extrait de l'exemple 1, protocole 3<br>Kawaïne<br>Hydroxypropylcellulose (Klucel G® de la société Hercules)<br>Alcool éthylique qsp | 15,000 g<br>2,000 g<br>3,500 g<br>100,000 g |

On applique cette lotion épaissie sur le cuir chevelu, une à deux fois par jour, à raison d'1 ml par application.

| Lotion niosomée : | |
|---|---|
| Extrait de l'exemple 1, protocole 1<br>Chimexane NL®<br>Cholestérol<br>Stéaroylglutamate monosodique<br>Conservateurs qs<br>Colorants qs<br>Parfum qs<br>Eau déminéralisée qsp | 1,000 g<br>0,475 g<br>0,475 g<br>0,050 g<br><br><br><br>100,000 g |

On applique cette lotion sur le cuir chevelu, une à deux fois par jour, à raison d'1 ml par application.

| Lotion : | |
|---|---|
| Extrait de l'exemple 1, protocole 2<br>Tyrosine<br>Monométhyléther de propylèneglycol (Dowanol PM® de Dow Chemical)<br>Alcool éthylique<br>Minoxidil<br>Eau qsp | 2,000 g<br>1,000 g<br>20,000 g Hydro<br>40,000 g<br>2,000 g<br>100,000 g |

On applique cette lotion épaissie sur le cuir chevelu, une à deux fois par jour, à raison d'1 ml par application.

## Revendications

1. Utilisation, à titre de principe actif, pour la préparation d'une composition cosmétique, d'une quantité suffisante d'au moins un extrait d'au moins un végétal du genre Chrysanthemum, pour augmenter la pigmentation de la peau et/ou des cheveux.

2. Utilisation, à titre de principe actif, pour la préparation d'une composition pharmaceutique, d'une quantité suffisante d'au moins un extrait d'au moins un végétal du genre Chrysanthemum, pour augmenter la pigmentation de la peau et/ou des cheveux.

3. Utilisation, à titre de principe actif, pour la préparation d'une composition cosmétique, d'une quantité suffisante d'au moins un extrait d'au moins un végétal du genre Chrysanthemum pour stimuler la mélanogenèse.

4. Utilisation, à titre de principe actif, pour la préparation d'une composition pharmaceutique, d'une quantité suffisante d'au moins un extrait d'au moins un végétal du genre Chrysanthemum pour stimuler la mélanogenèse.

5. Utilisation selon l'une quelconque des revendications précédentes, **caractérisée par le fait que** ledit végétal est choisi parmi les espèces *Chrysanthemum hortorum, Chrysanthemum morifolium, Chrysanthemum coronarium, Chrysanthemum myconis, Chrysanthemum sagitum, Chrysanthemum indicum,* ou encore *Chrysanthemum segetum*

6. Utilisation selon la revendication précédente, **caractérisée par le fait que** ledit végétal provient de l'espèce *Chrysanthemum morifolium*.

7. Utilisation selon la revendication 6, **caractérisée par le fait que** ledit végétal provient de la variété *sinense*.

8. Utilisation selon l'une quelconque des revendications précédentes, **caractérisée par le fait que** l'extrait d'au moins un végétal du genre Chrysanthemum est obtenu à partir de matériel végétal issu de plante entière cultivée *in vivo* ou issu de culture *in vitro*.

9. Utilisation selon l'une quelconque des revendications précédentes, **caractérisée par le fait que** l'extrait d'au moins un végétal du genre Chrysanthemum est obtenu à partir de feuilles, de fleurs, de tiges, de racines ou de cellules indifférenciées.

10. Utilisation selon l'une quelconque des revendications précédentes, **caractérisée par le fait que** l'extrait d'au moins un végétal du genre Chrysanthemum est en une quantité représentant de 0,01% à 15% du poids total de la composition et préférentiellement en une quantité représentant de 1% à 5% du poids total de la composition.

## Patentansprüche

1. Verwendung mindestens eines Extrakts mindestens einer Pflanze der Gattung Chrysanthemum als Wirkstoff zur Herstellung einer kosmetischen Zusammensetzung in einer Menge, die ausreichend ist, um die Pigmentierung der Haut und/oder der Haare zu fördern.

2. Verwendung mindestens eines Extrakts mindestens einer Pflanze der Gattung Chrysanthemum als Wirkstoff zur Herstellung einer pharmazeutischen Zusammensetzung in einer Menge, die ausreichend ist, um die Pigmentierung der Haut und/oder der Haare zu fördern.

3. Verwendung mindestens eines Extrakts mindestens einer Pflanze der Gattung Chrysanthemum als Wirkstoff zur Herstellung einer kosmetischen Zusammensetzung in einer Menge, die ausreichend ist, um die Melanogenese zu stimulieren.

4. Verwendung mindestens eines Extrakts mindestens einer Pflanze der Gattung Chrysanthemum als Wirkstoff zur Herstellung einer pharmazeutischen Zusammensetzung in einer Menge, die ausreichend ist, um die Melanogenese zu stimulieren.

5. Verwendung nach einem der vorhergehenden Ansprüche, **dadurch gekennzeichnet, daß** die Pflanze unter den

Arten *Chrysanthemum hortorum*, *Chrysanthemum morifolium, Chrysanthemum coronarium, Chry santhemum myconis, Chrysanthemum sagitum, Chrysanthemum indicum* oder *Chrysanthemum segetum* ausgewählt ist.

**6.** Verwendung nach dem vorhergehenden Anspruch, **dadurch gekennzeichnet, daß** die Pflanze aus der Art *Chrysanthemum morifolium* stammt.

**7.** Verwendung nach Anspruch 6, **dadurch gekennzeichnet, daß** die Pflanze aus der Varietät *sinense* stammt.

**8.** Verwendung nach einem der vorhergehenden Ansprüche, **dadurch gekennzeichnet, daß** der Extrakt mindestens einer Pflanze der Gattung Chrysanthemum aus Pflanzenmaterial erhalten wird, das von beliebigen Teilen einer *in-vivo* kultivierten Pflanze oder aus einer *in-vitro* Kultur stammt.

**9.** Verwendung nach einem der vorhergehenden Ansprüche, **dadurch gekennzeichnet, daß** der Extrakt mindestens einer Pflanze der Gattung Chrysanthemum ausgehend von Blättern, Blüten, Stengeln, Wurzeln oder undifferenzierten Zellen hergestellt wird.

**10.** Verwendung nach einem der vorhergehenden Ansprüche, **dadurch gekennzeichnet, daß** der Extrakt mindestens einer Pflanze der Gattung Chrysanthemum in einer Menge von 0,01 bis 15 % des Gesamtgewichts der Zusammensetzung und vorzugsweise 1 bis 5 % des Gesamtgewichts der Zusammensetzung verwendet wird.

## Claims

**1.** Use, as active principle, for the preparation of a cosmetic composition, of a sufficient quantity of at least one extract of at least one plant of the genus Chrysanthemum, in order to increase the pigmentation of the skin and/or the hair.

**2.** Use, as active principle, for the preparation of a pharmaceutical composition, of a sufficient quantity of at least one extract of at least one plant of the genus Chrysanthemum, in order to increase the pigmentation of the skin and/or the hair.

**3.** Use, as active principle, for the preparation of a cosmetic composition, of a sufficient quantity of at least one extract of at least one plant of the genus Chrysanthemum to stimulate melanogenesis.

**4.** Use, as active principle, for the preparation of a pharmaceutical composition, of a sufficient quantity of at least one extract of at least one plant of the genus Chrysanthemum to stimulate melanogenesis.

**5.** Use according to any one of the preceding claims, **characterized by** the fact that the said plant is selected from the species *Chrysanthemum hortorum, Chrysanthemum morifolium, Chrysanthemum coronarium, Chrysanthemum myconis, Chrysanthemum sagitum, Chrysanthemum indicum,* or alternatively *Chrysanthemum segetum.*

**6.** Use according to the preceding claim, **characterized by** the fact that the said plant comes from the species *Chrysanthemum morifolium.*

**7.** Use according to Claim 6, **characterized by** the fact that the said plant comes from the variety sinense.

**8.** Use according to any one of the preceding claims, **characterized by** the fact that the extract of at least one plant of the genus Chrysanthemum is obtained from plant material originating from the entire plant cultivated *in vivo* or originating from *in vitro* culture.

**9.** Use according to any one of the preceding claims, **characterized by** the fact that the extract of at least one plant of the genus Chrysanthemum is obtained from leaves, flowers, stems, roots or undifferentiated cells.

**10.** Use according to any one of the preceding claims, **characterized by** the fact that the extract of at least one plant of the genus Chrysanthemum is in a quantity representing from 0.01% to 15% of the total weight of the composition and preferentially in a quantity representing from 1% to 5% of the total weight of the composition.